# EUROPÄISCHE PATENTSCHRIFT

(11) **EP 0 916 653 B1**
(45) Veröffentlichungstag und Bekanntmachung des Hinweises auf die Patenterteilung: **09.01.2002**
(21) Anmeldenummer: 98120478.7
(22) Anmeldetag: 29.10.1998
(51) Int. Cl.: C07C 303/22, C07C 309/58

(54) **Verfahren zur Herstellung von 4-Nitro-2-sulfo-benzoesäure**
Process for the preparation of 4-nitro-2-sulphobenzoic acid
Procédé pour la préparation de l'acide 4-nitro-2-sulfobenzoique

(30) Priorität: 11.11.1997 DE 19749723
(43) Veröffentlichungstag der Anmeldung: 19.05.1999
(73) Patentinhaber: Bayer Aktiengesellschaft, 51368 Leverkusen (DE)
(72) Erfinder: Neumann, Karl Heinz Dr., 53757 Sankt Augustin (DE); Fiege, Helmut Dr., 51373 Leverkusen (DE)

(56) Entgegenhaltungen:
- US-A- 4 608 205
- M. HOLLEMAN: "L'action du cyanure de potassium sur le sel de potassium de l'acide métanitrobenzènesulfonique" RECUEIL DES TRAVAUX CHIMIQUES DES PAYS-BAS, Bd. 24, 1905, Seiten 194-208, XP002093686 Amsterdam, NL
- CHEMICAL ABSTRACTS, vol. 80, no. 9, 4. März 1974 Columbus, Ohio, US; abstract no. 47649k, Seite 332; XP002093687 & CS 150 000 A (D. HLAVACOVA, ET AL.)

## Beschreibung

Die vorliegende Erfindung betrifft ein vorteilhaftes Verfahren zur Herstellung von 4-Nitro-2-sulfo-benzoesäure durch Oxidation von 4-Nitro-toluol-2-sulfonsäure mit Salpetersäure.

4-Nitro-2-sulfo-benzoesäure ist ein wichtiges Zwischenprodukt beispielsweise zur Herstellung von Phenylsulfonylharnstoff-Herbiziden (siehe DE-A 4 236 902 und DE-A 4 230 933) und zur Herstellung von pH-Indikatoren (siehe DE-A 4 334 949).

Es ist bekannt, 4-Nitro-2-sulfo-benzoesäure durch Oxidation von 4-Nitro-toluol-2-sulfonsäure mit Kaliumpermanganat bei Temperaturen von Raumtemperatur bis ca. 95°C herzustellen (siehe Beilstein RN: 2 814 255 und DE-A 42 36 902). Der Einsatz von Kaliumpermanganat in technischem Maßstab verursacht jedoch unvertretbar hohen Aufwand für die Entsorgung des daraus entstehenden Braunsteins oder dessen Wiederaufarbeitung zu Kaliumpermanganat.

Man kann 4-Nitro-toluol-2-sulfonsäure auch mit Natriumhypochlorit an einem Nickelperoxid-Katalysator oxidieren (siehe JP-OS 57-200353). Dieses Verfahren wird bei 60 bis 85°C durchgeführt und erfordert hohe Katalysatormengen (9 Gew.-% bezogen auf das Edukt). Nickelverbindungen gelten jedoch als gefährliche Arbeitsstoffe (siehe Römpp Lexikon Chemie auf CD-ROM Version 1.2) und erfordern deshalb für eine sichere Handhabung und Entsorgung ebenfalls hohen Aufwand.

Bei der Oxidation von 4-Nitro-toluol-2-sulfonsäure besteht auch noch ein grundsätzliches Problem, weil ausgehend von wasserfeuchter 4-Nitro-toluol-2-sulfonsäure bei der Stufen-Differentialthermoanalyse bei 135°C eine starke autokatalytisch verlaufende Zersetzung und bei der Übersichts-Differentialthermoanalyse (Einzelheiten siehe Beispiel 5) bei 170°C eine exotherme Zersetzung festgestellt wurde. Der Fachmann erwartet aufgrund von diesen, an der wasserfeuchten Substanz bestimmten Daten, daß 4-Nitro-toluol-2-sulfonsäure enthaltende Reaktionsgemische bei Temperaturen über ca. 120°C unkontrollierbar reagieren können. Für Oxidationsreaktionen an 4-Nitro-toluol-2-sulfonsäure wird der Fachmann deshalb Temperaturen über 120°C zu vermeiden suchen.

Bei 2-Nitro-toluol-4-sulfonsäure, einem Isomeren der 4-Nitro-toluol-2-sulfonsäure, ist bekannt, daß man diese mit siedender Salpetersäure einer Dichte von 1,35 g/ml zu 4-Nitro-2-sulfo-benzoesäure oxidieren kann (siehe Rec. Trav. Chim. Pays-Bas 24, 208 (1905)). Salpetersäure einer Dichte von 1,35 g/ml weist einen Siedepunkt von ungefähr 120°C auf.

Man kann diese Reaktion auch so durchführen, daß man dem bei der Herstellung von 2-Nitro-toluol-4-sulfonsäure durch Sulfierung von o-Nitrotoluol anfallenden Reaktionsgemisch zunächst Wasser und eine Vanadium (V)-Verbindung zufügt, dann bei 130 bis 170°C 30 bis 100 gew.-%ige wäßrige Schwefelsäure zudosiert und gleichzeitig eine verdünntere Salpetersäure abdestilliert (siehe EP-A 460 544).

Da die Übersichts-Differentialthermoanalyse ausgehend von wasserfeuchter 2-Nitrotoluol-4-sulfonsäure erst bei 270°C eine exotherme Zersetzung anzeigt bestehen keine Bedenken, die vorgenannten Oxidationen von 2-Nitro-toluol-4-sulfonsäure im Temperaturbereich 120 bis 170°C durchzuführen. Wie oben aufgezeigt bestehen aber sehr wohl Bedenken, bei der Oxidation von 4-Nitro-toluol-2-sulfonsäure in diesem Temperaturbereich zu arbeiten.

Von Interesse ist auch, daß gemäß dem Stand der Technik eine Arbeitsweise unter Druck bisher nicht in Betracht gezogen wurde.

Es wurde nun ein Verfahren zur Herstellung von 4-Nitro-2-sulfo-benzoesäure durch Oxidation von 4-Nitro-toluol-2-sulfonsäure gefunden, das dadurch gekennzeichnet ist, daß man 4-Nitro-toluol-2-sulfonsäure in einem wäßrigen System bei 120 bis 170°C und bei einem Druck von bis zu 12 bar mit Salpetersäure umsetzt.

Die 4-Nitro-toluol-2-sulfonsäure muß nicht in reiner und trockener Form eingesetzt werden. Beispielsweise kann man den bei der Herstellung von 4-Nitro-toluol-2-sulfonsäure anfallenden, wasserfeuchten Filterkuchen verwenden.

Das zur Durchführung des erfindungsgemäßen Verfahrens erforderliche wäßrige System kann beispielsweise ein Gemisch aus Wasser und 4-Nitro-toluol-2-sulfonsäure sein, das 20 bis 70 Gew.-%, vorzugsweise 30 bis 55 Gew.-%, 4-Nitro-toluol-2-sulfonsäure enthalten kann.

Bevozugte Reaktionstemperaturen sind solche von 130 bis 165°C.

Der Druck beim erfindungsgemäßen Verfahren liegt beispielsweise im Bereich 1,5 bis 12 bar, vorzugsweise im Bereich 2 bis 10 bar. Es ist weiterhin bevorzugt, die erfindungsgemäße Oxidation bei dem Druck zu beginnen, der bei gegebener Reaktionstemperatur dem Dampfdruck von Wasser entspricht. Während der Oxidation steigt der Druck durch freiwerdende Stickoxide an. Er ist dann über ein Druckhalteventil zu kontrollieren, durch das Stickoxide im erforderlichen Umfang entweichen können.

Die eingesetzte Salpetersäure kann beispielsweise 30 bis 100 gew.-%ige wäßrige Salpetersäure sein. Vorzugsweise ist sie 55 bis 80 gew.-%ig.

Bezogen auf ein Mol 4-Nitro-toluol-2-sulfonsäure kann man beispielsweise 3 bis 8 Mol Salpetersäure einsetzen. Vorzugsweise beträgt diese Menge 4 bis 7 Mol.

Bei einer bevorzugten Ausführungsform der vorliegenden Erfindung verfährt man wie folgt: Man legt ein Gemisch aus Wasser und 4-Nitro-toluol-2-sulfonsäure in einem Autoklaven vor, verschließt ihn, erhitzt ihn auf Reaktionstemperatur und dosiert dann im Verlauf von beispielsweise 3 bis 8 Stunden die Salpetersäure dazu. Dabei wird der Druck kontrolliert und werden die Stickoxide im erforderlichen Umfang aus dem Autoklaven entweichen gelassen. Nach Beendigung der Salpetersäurezugabe kann man noch beispielsweise 0,5 bis 20 Stunden bei 120 bis 165°C nachrühren.

Bei einer weiteren bevorzugten Ausführungsform der vorliegenden Erfindung führt man dem Reaktionsgefäß vor und/oder während der Salpetersäurezugabe Sauerstoff oder ein Sauerstoff enthaltendes Gas zu.

Nach beendigter Oxidation und gegebenenfalls dem Ablauf einer Nachrührzeit kann man das Reaktionsgemisch abkühlen, das Reaktionsgefäß entspannen und restliche Stickoxide ausblasen, z.B. mit Stickstoff.

Die Isolierung der erfindungsgemäß hergestellten 4-Nitro-2-sulfo-benzoesäure kann dann beispielsweise durch Zugabe einer wäßrigen, vorzugsweise gesättigten Kaliumchlorid-Lösung und Filtration des dann ausfallenden Monohydrats des Kaliumsalzes der 4-Nitro-2-sulfo-benzoesäure erfolgen. Nach dessen Trocknung kann man schließlich im allgemeinen ein Produkt mit einer Reinheit von über 95 Gew.-% (bestimmt durch HPLC) erhalten.

Man kann die Fällung des Kaliumsalzes auch so vornehmen, daß man eine wäßrige Kaliumhydroxidlösung, beispielsweise einer Konzentration von 30 bis 50 Gew.-%, zugibt bis ein pH-Wert im Bereich von beispielsweise 0,5 bis 2,5 vorliegt. Der größte Teil der 4-Nitro-2-sulfo-benzoesäure fällt dann als Kaliumsalz aus. Gegebenenfalls kann man die Fällung durch Zugabe einer wäßrigen, vorzugsweise gesättigten Kaliumchlorid-Lösung vervollständigen.

Es ist überraschend, daß die erfindungsgemäße Oxidation kontrolliert durchgeführt werden kann, denn es hat sich gezeigt, daß ein typisches Gemisch aus 25 Gew.-% wasserfeuchter 4-Nitro-toluol-2-sulfonsäure, 46 Gew.-% 70 gew.-%iger wäßriger Salpetersäure und 29 Gew.-% Wasser in V4A-Stahl bei 170°C länger als 24 Stunden stabil ist und bei der Langzeit-Übersichts-Differentialthermoanalyse erst über 200°C eine exotherme Zersetzung eintritt. Man kann also erfindungsgemäß auf technisch unbedenkliche Weise die Nachteile vermeiden, die bei den Verfahren des Standes der Technik durch den Einsatz von Kaliumpermanganat oder Nickelperoxid auftreten.

Es sei auch noch auf das Vergleichsbeispiel (siehe Beispiel 3) hingewiesen, das zeigt, daß man bei der Oxidation von 4-Nitro-toluol-2-sulfonsäure in Bereichen über 120°C zu erwartenden Probleme nicht einfach durch eine Arbeitsweise bei tieferer Temperatur ausschalten kann.

### Beispiele

### Beispiel 1

In einem 1 l-Autoklaven aus Tantal mit Rührer und Rückflußkühler mit aufgesetztem auf 5 bar eingestelltem Druckhalteventil wurden 108,6 g wasserfeuchte 4-Nitrotoluol-2-sulfonsäure (Wassergehalt 22,5 Gew.-%) in 120 g Wasser suspendiert und auf 140°C erhitzt. Dabei baute sich ein Druck von 3,6 bar auf. Dann wurden 180 g 70 gew.-%ige wäßrige Salpetersäure innerhalb von 5 Stunden in den Autoklaven gepumpt. Der Druck stieg aufgrund der Stickoxid-Entwicklung bis auf die vorgegebenen 5 bar an und die Stickoxide wurden über das Druckhalteventil abgeblasen. Nach beendeter Salpetersäuredosierung wurde noch 12 Stunden bei 140°C nachgerührt. In den letzten Stunden der Nachrührzeit sank der Druck wieder auf weniger als 5 bar ab und der Abgasstrom versiegte. Nachdem der Autoklav auf Raumtemperatur abgekühlt worden war, wurde entspannt, und restliche Stickoxide wurden mit Stickstoff ausgeblasen. Der Autoklaveninhalt wurde in einen Kolben mit Rührer überführt und 150 g kalt gesättigte wäßrige Kaliumchlorid-Lösung zugegeben. Die 4-Nitro-2-sulfo-benzoesäure fiel als Kaliumsalz-monohydrat aus, wurde abfiltriert, mit kaltem Wasser gewaschen und im Trockenschrank getrocknet. Es wurden 96 g 4-Nitro-2-sulfo-benzoesäure in Form des Monohydrats des Kaliumsalzes mit einem Gehalt von 97 Gew.-% (HPLC) isoliert.

### Beispiel 2

Es wurde gearbeitet wie in Beispiel 1, jedoch wurde das Druckhalteventil auf 9 bar eingestellt, das Gemisch aus 4-Nitro-toluol-2-sulfonsäure und Wasser auf 160°C erhitzt, die Salpetersäure im Verlauf von 4 Stunden zudosiert und nach Beendigung der Salpetersäuredosierung nur 1 Stunde bei 160°C nachgerührt. Es wurden 92,4 g 4-Nitro-2-sulfo-benzoesäure in Form des Kaliumsalz-Monohydrates mit einem Gehalt von 97 Gew.-% (HPLC) isoliert.

### Beispiel 3 (Zum Vergleich)

Eine Suspension aus einer 108,6 g wasserfeuchten 4-Nitro-toluol-2-sulfonsäure (Wassergehalt 22,5 Gew.-%) und 120 ml Wasser wurden in einem Kolben mit Rückflußkühler und Rührer mit einem Ölbad auf 100°C Innentemperatur erwärmt. Nach dem Erreichen dieser Temperatur wurden unter Rückflußbedingungen im Verlauf von 4 Stunden 180 g 70 gew.-%ige wäßrige Salpetersäure zugetropft. Während der Salpetersäurezudosierung stieg die Temperatur des Reaktionsgemisches bis auf 113°C. Nach Beendigung der Zugabe der Salpetersäure wurde noch 3 Stunden unter Rückflußbedingungen nachgerührt. Danach wurde das Reaktionsgemisch auf Raumtemperatur abgekühlt und mit einer kalt gesättigten wäßrigen Kaliumchlorid-Lösung versetzt. Der entstandene Rückstand wurde abfiltriert und getrocknet. Die HPLC-Analyse des Rückstands ergab, daß es sich dabei zu über 99 Gew.-% um das Kaliumsalz der 4-Nitro-toluol-2-sulfonsäure handelte. Es hatte also praktisch keine Umsetzung stattgefunden.

### Beispiel 4

In der in Beispiel 1 beschriebenen Apparatur wurden 181 g wasserfeuchte 4-Nitrotoluol-2-sulfonsäure (enthaltend 22,5 Gew.-% Wasser) und 200 g Wasser vorgelegt und unter Rühren auf 140°C erhitzt. Nach Erreichen der vorgegebenen Temperatur wurden in 5 Stunden 300 g 70 gew.-%ige Salpetersäure in den Reaktor gepumpt. Der Druck stieg auf Grund der Stickoxidentwicklung bis auf die vorgegebenen 5 bar an, und die Stickoxide wurden über das Druckhaltenventil abgeblasen. Nach beendeter Dosierung wurden weitere 8 Stunden bei 140°C nachgerührt. Danach wurde die Temperatur auf 160°C erhöht und weitere 4 Stunden bei dieser Temperatur gerührt. Der Ansatz wurde auf Raumtemperatur abgekühlt und noch vorhandene Stickoxide mit Stickstoff ausgeblasen. Der Autoklav wurde geöffnet und der Inhalt in einen Kolben überführt. Unter gutem Rühren wurde bei Raumtemperatur durch Zugabe 50 gew.- %iger wäßriger Kalilauge ein pH-Wert von 1,8 eingestellt. Zusätzlich wurde gesättigte Kaliumchlorid-Lösung zur Vervollständigung des Niederschlags zugegeben und die Kristallsuspension 2 Stunden bei 10°C gerührt. Das Produkt wurde abgesaugt, 3 mal mit je 50 ml kaltem Wasser gewaschen und im Vakuumtrockenschrank getrocknet. Man erhielt 164 g 4-Nitro-benzoesäure-2-kaliumsulfonat-mono-hydrat mit einem Gehalt von 97,5% (HPLC).

### Beispiel 5

Durchführung von Übersichts-Differentialthermoanalysen.

Die Probe wurde in einen Tiegel aus V4A-Stahl eingewogen, der Tiegel verschlossen und die Differential-Thermoanalyse in einem Gerät der Fa. Mettler, Typ TC 11/ DSC 25, durchgeführt. Die Temperatur wurde von 30 auf 250°C mit einer Steigerungsrate von 1 K/min erhöht.

Als wasserfeuchte 4-Nitro-toluol-2-sulfonsäure wurde solche mit einem Wassergehalt von 22,5 Gew.-% eingesetzt.

Durchführung von Langzeit-Übersichts-Differentialthermoanalysen.

Es wurde gearbeitet wie oben beschrieben, jedoch wurde von 30 bis 170°C mit einer Steigerungsrate von 3 K/min erhitzt, dann 24 Stunden bei 170°C getempert und anschließend mit der gleichen Steigerungsrate bis auf 250°C weiter erhitzt.

## Patentansprüche

1. Verfahren zur Herstellung von 4-Nitro-2-sulfo-benzoesäure durch Oxidation von 4-Nitro-toluol-2-sulfonsäure, **dadurch gekennzeichnet, daß** man 4-Nitrotoluol-2-sulfonsäure in einem wäßrigen System bei 120 bis 170°C und bei einem Druck von bis zu 12 bar mit Salpetersäure umsetzt.

2. Verfahren nach Anspruch 1, **dadurch gekennzeichnet, daß** man die Umsetzung bei 130 bis 165°C vornimmt.

3. Verfahren nach Ansprüchen 1 und 2, **dadurch gekennzeichnet, daß** der Druck im Bereich 1,5 bis 12 bar liegt.

4. Verfahren nach Ansprüchen 1 bis 3, **dadurch gekennzeichnet, daß** das wäßrige System ein Gemisch aus Wasser und 4-Nitro-toluol-2-sulfonsäure ist, das 20 bis 70 Gew.-% 4-Nitro-toluol-2-sulfonsäure enthält.

5. Verfahren nach Ansprüchen 1 bis 4, **dadurch gekennzeichnet, daß** die Salpetersäure in Form einer 30 bis 100 gew.-%igen wäßrigen Salpetersäure eingesetzt wird.

6. Verfahren nach Ansprüchen 1 bis 5, **dadurch gekennzeichnet, daß** man bezogen auf ein Mol 4-Nitro-toluol-2-sulfonsäure 3 bis 8 Mol Salpetersäure einsetzt.

7. Verfahren nach Ansprüchen 1 bis 6, **dadurch gekennzeichnet, daß** man ein Gemisch aus Wasser und 4-Nitro-toluol-2-sulfonsäure in einem Autoklaven vorlegt, ihn verschließt, ihn auf Reaktionstemperatur erhitzt und dann die Salpetersäure dazu zudosiert, wobei man den Druck kontrolliert und Stickoxide im erforderlichen Umfang aus dem Autoklaven entweichen läßt.

8. Verfahren nach Ansprüchen 1 bis 7, **dadurch gekennzeichnet, daß** man vor und/oder während der Salpetersäurezugabe Sauerstoff oder ein Sauerstoff enthaltendes Gas dem Reaktionsgefäß zuführt.

9. Verfahren nach Ansprüchen 1 bis 8, **dadurch gekennzeichnet, daß** man aus dem Reaktionsgemisch die hergestellte 4-Nitro-2-sulfo-benzoesäure durch Zugabe einer wäßrigen Kaliumchlorid- oder Kaliumhydroxid-Lösung und Filtration des dann ausfallenden Monohydrats des Kaliumsalzes der 4-Nitro-2-sulfo-benzoesäure isoliert.

10. Verfahren nach Anspruch 9, **dadurch gekennzeichnet, daß** man eine wäßrige Kaliumhydroxid-Lösung und anschließend eine wäßrige Kaliumchlorid-Lösung zugibt.

## Claims

1. Process for preparing 4-nitro-2-sulpho-benzoic acid by oxidation of 4-nitrotoluene-2-sulphonic acid, **characterized in that** 4-nitro-toluene-2-sulphonic acid is reacted with nitric acid in an aqueous system at 120 to 170°C and at a pressure of up to 12 bar.

2. Process according to Claim 1, **characterized in that** the reaction is carried out at 130 to 165°C.

3. Process according to Claims 1 and 2, **characterized in that** the pressure is in the range of from 1.5 to 12 bar.

4. Process according to Claims 1 to 3, **characterized in that** the aqueous system is a mixture of water and 4-nitro-toluene-2-sulphonic acid which comprises 20 to 70% by weight of 4-nitro-toluene-2-sulphonic acid.

5. Process according to Claims 1 to 4, **characterized in that** the nitric acid is employed in the form of a 30 to 100% strength by weight aqueous nitric acid.

6. Process according to Claims 1 to 5, **characterized in that** 3 to 8 mol of nitric acid are employed per mole of 4-nitro-toluene-2-sulphonic acid.

7. Process according to Claims 1 to 6, **characterized in that** a mixture of water and 4-nitro-toluene-2-sulphonic acid is initially charged in an autoclave which is then closed and heated to reaction temperature, and the nitric acid is then metered in and the pressure is monitored and nitrogen oxides are allowed to escape from the autoclave to the extent required.

8. Process according to Claims 1 to 7, **characterized in that** prior to and/or during the addition of nitric acid oxygen or an oxygen-containing gas is introduced into the reaction vessel.

9. Process according to Claims 1 to 8, **characterized in that** the 4-nitro-2-sulpho-benzoic acid which is prepared is isolated from the reaction mixture by addition of an aqueous potassium chloride or potassium hydroxide solution and filtration of the monohydrate of the potassium salt of 4-nitro-2-sulpho-benzoic acid which then precipitates.

10. Process according to Claims 1 to 9, **characterized in that** an aqueous potassium hydroxide solution and subsequently an aqueous potassium chloride solution are added.

## Revendications

1. Procédé de préparation d'acide 4-nitro-2-sulfobenzoïque par oxydation de l'acide 4-nitrotoluène-2-sulfonique, qui est **caractérisé en ce que** l'on fait réagir l'acide 4-nitrotoluène-2-sulfonique avec de l'acide nitrique, dans un système aqueux, à une température allant de 120 à 170°C et sous une pression allant jusqu'à 12 bar.

2. Procédé suivant la revendication 1, **caractérisé en ce que** l'on exécute la réaction à une température allant de 130 à 165°C.

3. Procédé suivant les revendications 1 et 2, **caractérisé en ce que** la pression se situe dans l'intervalle allant de 1,5 à 12 bar.

4. Procédé suivant les revendications 1 à 3, **caractérisé en ce que** le système aqueux est un mélange d'eau et d'acide 4-nitrotoluène-2-sulfonique, qui contient de 20 à 70% en poids d'acide 4-nitrotoluène-2-sulfonique.

5. Procédé suivant les revendications 1 à 4, **caractérisé en ce que** l'acide nitrique est mis en oeuvre sous la forme d'un acide nitrique aqueux de 30 à 100% en poids.

6. Procédé suivant les revendications 1 à 5, **caractérisé en ce que** sur base d'une mole d'acide 4-nitrotoluène-2-sulfonique, on met en oeuvre 3 à 8 moles d'acide nitrique.

7. Procédé suivant les revendications 1 à 6, **caractérisé en ce que** l'on dispose un mélange d'eau et d'acide 4-nitrotoluène-2-sulfonique dans un autoclave, on le ferme, on le chauffe jusqu'à la température de réaction et on y ajoute l'acide nitrique, la pression étant contrôlée et l'oxyde d'azote pouvant s'échapper de l'autoclave, dans les limites nécessaires.

8. Procédé suivant les revendications 1 à 7, **caractérisé en ce que** l'on amène dans le récipient de réaction, avant et/ou pendant l'addition de l'acide nitrique, de l'oxygène ou un gaz contenant de l'oxygène.

9. Procédé suivant les revendications 1 à 8, **caractérisé en ce que** l'on isole du mélange réactionnel, l'acide 4-nitro-2-sulfobenzoïque préparé par addition d'une solution aqueuse de chlorure de potassium ou d'hydroxyde de potassium et filtration du monohydrate du sel de potassium de l'acide 4-nitro-2-sulfobenzoïque alors précipité.

10. Procédé suivant la revendication 9, **caractérisé en ce que** l'on ajoute une solution aqueuse d'hydroxyde de potassium et ensuite, une solution aqueuse de chlorure de potassium.
